# EUROPÄISCHE PATENTANMELDUNG

(11) **EP 4 378 428 A1**
(43) Veröffentlichungstag der Anmeldung: **05.06.2024**
(21) Anmeldenummer: 23207335.3
(22) Anmeldetag: 02.11.2023
(51) Int. Cl.: A61F 2/38, A61F 2/30

(54) **KNIEGELENKENDOPROTHESE**

(30) Priorität: 29.11.2022 DE 102022212768
(71) Anmelder: Aesculap AG, 78532 Tuttlingen (DE)
(72) Erfinder: Kempf, Harry, 72510 Stetten a. k. M. (DE)
(74) Vertreter: Patentanwälte Ruff, Wilhelm, Beier, Dauster & Partner mbB

(57) **Zusammenfassung**

Eine derartige Kniegelenkendoprothese, aufweisend eine Tibiakomponente mit einem distalen Befestigungsabschnitt, der zur Befestigung an einer proximalen Tibia eingerichtet ist, und mit einem proximalen Verbindungsabschnitt, und aufweisend eine Meniskuskomponente mit einer proximalen Gleitfläche, die zur Artikulation mit einer femoralen Gelenkkomponente eingerichtet ist, und mit einem distalen Gegenverbindungsabschnitt, der zum Ausbilden einer Fügeverbindung mit dem proximalen Verbindungsabschnitt der Tibiakomponente eingerichtet ist, ist bekannt.

Erfindungsgemäß ist eine Schraubverbindung vorhanden und zur zusätzlichen Fixierung der Fügeverbindung eingerichtet, wobei die Schraubverbindung wenigstens ein in eine Längsbohrung der Meniskuskomponente eingesetztes Schraubelement und wenigstens ein der Tibiakomponente zugeordnetes Gegenschraubelement aufweist, mit welchem das Schraubelement zum Ausbilden der Schraubverbindung verschraubbar ist.

## Beschreibung

Die Erfindung betrifft eine Kniegelenkendoprothese, aufweisend eine Tibiakomponente mit einem distalen Befestigungsabschnitt, der zur Befestigung an einer proximalen Tibia eingerichtet ist, und mit einem proximalen Verbindungsabschnitt, und aufweisend eine Meniskuskomponente mit einer proximalen Gleitfläche, die zur Artikulation mit einer femoralen Gelenkkomponente eingerichtet ist, und mit einem distalen Gegenverbindungsabschnitt, der zum Ausbilden einer Fügeverbindung mit dem proximalen Verbindungsabschnitt der Tibiakomponente eingerichtet ist.

Kniegelenkendoprothesen sind im Bereich der orthopädischen Chirurgie allgemein bekannt und dienen als künstlicher Ersatz für natürliche Kniegelenke. Übliche Kniegelenkendoprothesen weisen eine Tibiakomponente und eine Meniskuskomponente auf. Die Tibiakomponente ist zur Befestigung an einer proximalen Tibia des Patienten eingerichtet und weist zu diesem Zweck einen distalen Befestigungsabschnitt auf, der beispielsweise in Form eines intramedullären Befestigungsschafts gestaltet sein kann. Die Meniskuskomponente weist eine proximale Gleitfläche zur gleitbeweglichen Artikulation mit einer femoralen Gelenkkomponente auf. Bei der femoralen Gelenkkomponente kann es sich um den natürlichen distalen Femur, genauer: dessen Kondylenflächen, oder um eine prothetische Femurkomponente handeln. Bei der Implantation der Kniegelenkendoprothese wird die Meniskuskomponente mit der üblicherweise bereits an der proximalen Tibia befestigten Tibiakomponente verbunden. Zu diesem Zweck weist die Tibiakomponente einen proximalen Verbindungsabschnitt auf und die Meniskuskomponente weist einen komplementären distalen Gegenverbindungsabschnitt auf. Der Verbindungsabschnitt und der Gegenverbindungsabschnitt sind zum Ausbilden einer Fügeverbindung eingerichtet, bei der es sich üblicherweise um eine Steck-, Rast- und/oder Schnappverbindung handelt.

Aufgabe der Erfindung ist es, eine Kniegelenkendoprothese der eingangs genannten Art bereitzustellen, die gegenüber dem Stand der Technik verbesserte Eigenschaften aufweist.

Diese Aufgabe wird dadurch gelöst, dass eine Schraubverbindung vorhanden und zur zusätzlichen Fixierung der Fügeverbindung eingerichtet ist, wobei die Schraubverbindung wenigstens ein in eine Längsbohrung der Meniskuskomponente eingesetztes Schraubelement und wenigstens ein der Tibiakomponente zugeordnetes Gegenschraubelement aufweist, mit welchem das Schraubelement zum Ausbilden der Schraubverbindung verschraubbar ist. Durch die erfindungsgemäße Lösung wird eine zusätzliche Fixierung der Fügeverbindung erreicht.

Dies wirkt einem ungewollten Lösen der Fügeverbindung entgegen. Ein ungewolltes Lösen der Fügeverbindung im implantierten Zustand der Kniegelenkendoprothese beeinträchtigt deren Funktion, führt zu einer erheblichen Beeinträchtigung des Patienten und macht üblicherweise eine Revisionsoperation notwendig. Diese Nachteile werden durch die erfindungsgemäße zusätzliche Schraubverbindung zwischen der Meniskuskomponente und der Tibiakomponente überwunden. Die Schraubverbindung weist das wenigstens eine Schraubelement und das wenigstens eine Gegenschraubelement auf. Das Schraubelement und das Gegenschraubelement sind zueinander komplementär. Das Schraubelement ist der Meniskuskomponente zugeordnet. Das Gegenschraubelement ist der Tibiakomponente zugeordnet. Bei einer Ausgestaltung ist das Schraubelement eine Schraube und das Gegenschraubelement ist eine komplementäre Gewindebohrung. Dabei sind die Begriffe "Schraubverbindung", "Schraubelement", "Gegenschraubelement" und "verschraubt" nicht insoweit einschränkend auszulegen. Bei einer weiteren Ausgestaltung ist die Schraubverbindung als Bajonettverschlussverbindung gestaltet, wobei das Schraubelement und das Gegenschraubelement als zueinander komplementäre Bajonettverschlusselemente gestaltet sind. Die in die Meniskuskomponente eingebrachte Längsbohrung ist zur Aufnahme des Schraubelements eingerichtet. Die Längsbohrung ist axial entlang einer Bohrungsachse erstreckt, die wenigstens eine proximodistale Richtungskomponente aufweist. Bei einer Ausgestaltung weist die Bohrungsachse zudem eine anteroposteriore Richtungskomponente auf. Bei einer weiteren Ausgestaltung weist die Bohrungsachse alternativ oder zusätzlich eine mediolaterale Richtungskomponente auf. Mit anderen Worten ausgedrückt: Die Längsbohrung ist im weitesten Sinne von oben nach unten, d.h. zwischen einer proximalen Oberseite und einer distalen Unterseite der Meniskuskomponente, durch die Meniskuskomponente längserstreckt, wobei die Längserstreckung nicht notwendigerweise parallel oder gar koaxial zu einer proximodistalen Längsachse der Meniskuskomponente verlaufen muss. Vorzugsweise ist die Längsbohrung eine Durchgangsbohrung. Vorzugsweise ist die Tibiakomponente aus einem metallischen Werkstoff gefertigt. Vorzugsweise ist die Meniskuskomponente aus einem polymeren Werkstoff gefertigt. Vorzugsweise ist das Schraubelement aus einem metallischen Werkstoff gefertigt. Bei einer Ausgestaltung ist das Gegenschraubelement unmittelbar in die Tibiakomponente eingebracht und/oder durch einen Abschnitt der Tibiakomponente gebildet. Bei einer weiteren Ausgestaltung ist das Gegenschraubelement ein separates Bauteil und/oder durch einen Abschnitt des separaten Bauteils gebildet, wobei das separate Bauteil wenigstens im implantierten Zustand mit der Tibiakomponente verbunden ist.

Die in dieser Beschreibung verwendeten Lage- und Richtungsbezeichnungen beziehen sich auf den Körper eines Patienten und sind insoweit gemäß ihrer üblichen anatomischen Bedeutung zu bestehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximodistale Achse alternativ als Z-Achse bezeichnet werden. Die mediolaterale Achse kann als Y-Achse bezeichnet werden. Die anteroposteriore Achse kann als X-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet.

In Ausgestaltung der Erfindung ist die Längsbohrung relativ zu einer proximodistalen Längsachse der Meniskuskomponente in proximaler Richtung anterior geneigt. Dies bedeutet, dass die Bohrungsachse der Längsbohrung nach vorne geneigt längserstreckt ist. Hierdurch ist die Längsbohrung intraoperativ in verbesserter Weise zugänglich. Durch die anteriore Neigung wird vermieden, dass die femorale Gelenkkomponente die Längsbohrung verdeckt. Ein Operateur kann die Längsbohrung deshalb in verbesserter Weise erreichen, um beispielsweise das Schraubelement einzusetzen oder einen Werkzeugschaft zum Antreiben des Schraubelements anzusetzen.

In weiterer Ausgestaltung der Erfindung weist die Längsbohrung eine Einführöffnung zum Einführen des Schraubelements und/oder eines Werkzeugschafts zum Antreiben des Schraubelements auf. Die Einführöffnung ist einends der Längsbohrung angeordnet. Vorzugsweise ist die Einführöffnung proximal und/oder anterior an der Meniskuskomponente angeordnet. Die Einführöffnung erlaubt insbesondere eine nochmals vereinfachte Herstellung der Schraubverbindung.

In weiterer Ausgestaltung der Erfindung ist die Einführöffnung an einer anterioren Vorderseite der Meniskuskomponente angeordnet. Hierdurch wird vermieden, dass die femorale Gelenkkomponente die Einführöffnung abdeckt. Folglich ist die Einführöffnung auch während der Kniegelenkersatzoperation für den Operateur leicht einsehbar und zugänglich. Hierdurch kann der Operateur das Schraubelement und/oder den Werkzeugschaft in besonders einfacher Weise in die Einführöffnung einführen.

In weiterer Ausgestaltung der Erfindung ist die Einführöffnung relativ zu einem anterioren Rand der Gleitfläche in distaler Richtung nach unten versetzt angeordnet. Hierdurch ist die Einführöffnung für den Operateur in besonders einfacher Weise einsehbar und zugänglich. Durch die Anordnung unterhalb des anterioren Rands der Gleitfläche wird vermieden, dass die Einführöffnung von der femoralen Gelenkkomponente verdeckt wird. Hierdurch kann der Operateur den Werkzeugschaft zum Antreiben des Schraubelements oder das Schraubelement selbst in besonders einfacher Weise in die Einführöffnung einführen.

In weiterer Ausgestaltung der Erfindung ist das Schraubelement in einem von der Tibiakomponente getrennten Zustand der Meniskuskomponente verliersicher in der Längsbohrung gehalten. Die verliersichere Halterung stellt sicher, dass das Schraubelement nicht ungewollt aus der Längsbohrung herausfällt, während die Meniskuskomponente an der Tibiakomponente angebracht wird. Dies erlaubt eine besonders einfache und sichere Handhabung. Zudem wird die Patientensicherheit erhöht. Vorzugsweise ist wenigstens ein Kopfabschnitt des Schraubelements verliersicher in der Längsbohrung gehalten. Vorzugsweise weist der Kopfabschnitt eine eingesenkte Antriebsgeometrie zur Aufnahme einer komplementären Antriebsgeometrie eines Werkzeugschafts auf. Zwecks verliersicherer Halterung ist das Schraubelement vorzugsweise radial und/oder axial in der Längsbohrung gehalten. Die verliersichere Halterung des Schraubelements wird vorzugsweise bereits bei der Herstellung der Kniegelenkendoprothese etabliert. Folglich kann auch davon gesprochen werden, dass das Schraubelement verliersicher an der Meniskuskomponente vormontiert ist.

In weiterer Ausgestaltung der Erfindung weist die Längsbohrung einen ersten Bohrungsabschnitt, in welchem das Schraubelement verliersicher gehalten ist, und einen zweiten Bohrungsabschnitt auf, der einends die Einführöffnung aufweist und andernends in den ersten Bohrungsabschnitt mündet. Das Schraubelement, vorzugsweise dessen Kopfabschnitt, ist radial und/oder axial formschlüssig in dem ersten Bohrungsabschnitt gehalten. Vorzugsweise ist der erste Bohrungsabschnitt zu diesem Zweck maßlich auf das Schraubelement, vorzugsweise dessen Kopfabschnitt, abgestimmt. Bei dieser Ausgestaltung der Erfindung ist die Einführöffnung, vorzugsweise ausschließlich, zum Einführen des besagten Werkzeugschafts eingerichtet. Das Schraubelement wird vorzugsweise bereits bei der Fertigung der Kniegelenkendoprothese in den ersten Bohrungsabschnitt eingebracht und verliersicher vormontiert. Vorzugsweise mündet der erste Bohrungsabschnitt einends in den zweiten Bohrungsabschnitt. Andernends weist der erste Bohrungsabschnitt vorzugsweise eine Montageöffnung auf. Die verliersichere Montage des Schraubelements erfolgt vorzugsweise über die Montageöffnung. Vorzugsweise weist die Längsbohrung einends die Einführöffnung und andernends die Montageöffnung auf.

In weiterer Ausgestaltung der Erfindung ist ein Durchmesser der Einführöffnung kleiner als ein Durchmesser des ersten Bohrungsabschnitts. Dies ist eine besonders bevorzugte Ausgestaltung der Erfindung. Durch den in Relation verkleinerten Durchmesser der Einführöffnung werden Gewebeirritationen und damit einhergehende Beeinträchtigungen des Patienten in der Verwendung der Kniegelenkendoprothese vermieden. Der Durchmesser der Einführöffnung ist in Relation zu dem Durchmesser des ersten Bohrungsabschnitts und damit auch zum Durchmesser des Schraubelements, insbesondere dessen Kopfabschnitt, verkleinert. Folglich kann auch davon gesprochen werden, dass der verkleinerte Durchmesser der Einführöffnung zu einer wenigstens abschnittsweisen Abdeckung des Schraubelements, insbesondere des Kopfabschnitts, führt. Diese Abdeckung wirkt den besagten Gewebeirritationen entgegen.

In weiterer Ausgestaltung der Erfindung beträgt der Durchmesser der Einführöffnung weniger als 80 %, bevorzugt weniger als 60 %, besonders bevorzugt weniger als 50 %, des Durchmessers des ersten Bohrungsabschnitts. Die vorbezeichneten Werte haben sich als vorteilhaft erwiesen.

In weiterer Ausgestaltung der Erfindung ist der erste Bohrungsabschnitt mit einer, insbesondere in die Meniskuskomponente eingegossenen und/oder metallischen, Einsatzhülse versehen, in welcher das Schraubelement radial und axial formschlüssig gehalten ist. Das Schraubelement, vorzugsweise dessen Kopfabschnitt, ist entlang seiner Längsachse wenigstens eine in Richtung, bevorzugt in beide Richtungen der Längsachse, formschlüssig in der Einsatzhülse gehalten. Die Einsatzhülse erlaubt eine vereinfachte verliersichere Montage des Schraubelements an der Meniskuskomponente. Sofern die Einsatzhülse metallisch ist, kann eine besonders zuverlässige Übertragung der Kräfte der Schraubverbindung von dem Schraubelement auf die Meniskuskomponente erreicht werden. Dies ist insbesondere dann vorteilhaft, wenn die Meniskuskomponente aus einem polymeren Werkstoff gefertigt ist.

In weiterer Ausgestaltung der Erfindung ist das Gegenschraubelement eine Gewindebohrung. Die Gewindebohrung kann unmittelbar in die Tibiakomponente eingebracht sein. Alternativ kann die Gewindebohrung in ein separates Bauteil eingebracht sein, welches fest mit der Tibiakomponente verbunden ist. Die Gewindebohrung ist koaxial zu der der Längsbohrung erstreckt.

In weiterer Ausgestaltung der Erfindung ist die Gewindebohrung in den Befestigungsabschnitt der Tibiakomponente eingebracht und/oder koaxial zu einer proximodistalen Längsachse der Tibiakomponente längserstreckt. Dies erlaubt eine besonders einfache und kostengünstige Herstellung.

In weiterer Ausgestaltung der Erfindung ist die Gewindebohrung in eine in die Tibiakomponente, insbesondere den Befestigungsabschnitt, eingesetzte Gewindehülse eingebracht und/oder relativ zu einer proximodistalen Längsachse der Tibiakomponente geneigt. Diese Ausgestaltung erlaubt insbesondere dann eine vereinfachte Herstellung, wenn die Längsbohrung zu der proximodistalen Längsachse der Meniskuskomponente und/oder Tibiakomponente anterior geneigt ist. Eine hierzu koaxial geneigt längserstreckte Gewindebohrung ist nur mit relativ hohem Fertigungsaufwand unmittelbar in den Befestigungsabschnitt einzubringen. Die Gewindehülse erlaubt eine diesbezügliche Vereinfachung. Vorzugsweise weist der Befestigungsabschnitt eine Zentralbohrung auf, in welche die Gewindehülse fest eingesetzt ist. Die Zentralbohrung ist vorzugsweise parallel zu einer proximodistalen Längsachse der Tibiakomponente.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in perspektivischer Explosionsdarstellung eine Ausführungsform einer erfindungsgemäßen Kniegelenkendoprothese,
- Fig. 2: die Kniegelenkendoprothese nach Fig. 1 in einer schematischen und geringfügig perspektivischen Längsschnittdarstellung,
- Fig. 3: eine weitere Ausführungsform einer erfindungsgemäßen Kniegelenkendoprothese in einer schematischen Längsschnittdarstellung,
- Fig. 4: in schematischer Perspektivdarstellung eine weitere Ausführungsform einer erfindungsgemäßen Kniegelenkendoprothese,
- Fig. 5: die Kniegelenkendoprothese nach Fig. 4 in einer schematischen und geringfügig perspektivischen Längsschnittdarstellung, wobei einzelne Komponenten der Kniegelenkendoprothese noch nicht vollständig miteinander zusammengefügt sind, und
- Fig. 6: die Kniegelenkendoprothese nach den Fig. 4 und 5 in einer weiteren schematischen und geringfügig perspektivischen Längsschnittdarstellung zusammen mit einem schematisch angedeuteten Werkzeugschaft.

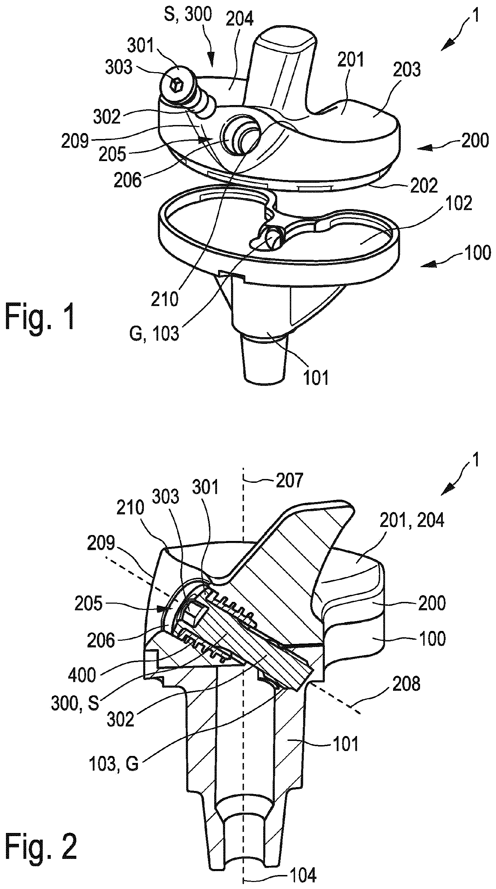

Gemäß den Fig. 1 und 2 ist eine Kniegelenkendoprothese 1 zur Verwendung bei einer Kniegelenkersatzoperation vorgesehen und weist eine Tibiakomponente 100 und eine Meniskuskomponente 200 auf.

Die Tibiakomponente 100 weist einen distalen Befestigungsabschnitt 101 und einen proximalen Verbindungsabschnitt 102 auf. Der distale Befestigungsabschnitt 101 ist zur Befestigung an einer proximalen Tibia eingerichtet. Der distale Befestigungsabschnitt 101 kann auf eine dem Fachmann bekannte Weise mit der proximalen Tibia verschraubt oder in diese einzementiert werden. Bei der gezeigten Ausführungsform ist der distale Befestigungsabschnitt in Form eines proximodistal längserstreckten Befestigungsschafts gestaltet. Der proximale Verbindungsabschnitt dient einer Verbindung mit der Meniskuskomponente 200. Die Tibiakomponente 100 kann auch als Tibiaplateau bezeichnet werden.

Die Meniskuskomponente 200 weist eine proximale Gleitfläche 201 auf. Die proximale Gleitfläche 201 ist zur Artikulation mit einer femoralen Gelenkkomponente eingerichtet. Bei der femoralen Gelenkkomponente kann es sich um eine prothetische Femurkomponente handeln, die Bestandteil der Kniegelenkendoprothese 1 sein kann. Alternativ und für den Fall, dass die Kniegelenkendoprothese 1 lediglich einen teilweisen Ersatz eines natürlichen Kniegelenks bildet, kann es sich bei der femoralen Gelenkkomponente um einen natürlichen distalen Femur handeln. Die proximale Gleitfläche 201 weist vorliegend zwei Gleitflächenabschnitte 203, 204 auf. Die Gleitflächenabschnitte 203, 204 sind mediolateral voneinander beabstandet und können auch als lateraler Gleitflächenabschnitt 203 und medialer Gleitflächenabschnitt 204 bezeichnet werden. Die Meniskuskomponente 200 weist zudem einen distalen Gegenverbindungsabschnitt 202 auf. Der distale Gegenverbindungsabschnitt 202 und der proximale Verbindungsabschnitt 102 sind zueinander komplementär und zum Ausbilden einer Fügeverbindung eingerichtet. Vorliegend kann der Gegenverbindungsabschnitt 202 mit dem Verbindungsabschnitt 102 verrastet werden, so dass es sich bei der besagten Fügeverbindung folglich um eine Rastverbindung handelt. Bei in den Figuren nicht gezeigten Ausführungsformen ist die Fügeverbindung eine Steck- und/oder Klemmverbindung. Weitere Details der Gestaltung und Funktion der Fügeverbindung sind im Hinblick auf die vorliegende Erfindung nicht wesentlich. Diesbezüglich weitere Erläuterungen sind daher entbehrlich.

Zur zusätzlichen Fixierung der Fügeverbindung zwischen dem Verbindungsabschnitt 102 und dem Gegenverbindungsabschnitt 202 ist eine Schraubverbindung vorhanden.

Die Schraubverbindung weist ein Schraubelement 300 und ein Gegenschraubelement 103 auf. Das Schraubelement 300 ist der Meniskuskomponente 200 zugeordnet. Das Gegenschraubelement 103 ist der Tibiakomponente 100 zugeordnet und bei der gezeigten Ausführungsform durch einen noch näher erläuterten Abschnitt der Tibiakomponente 100 gebildet. Bei weiteren Ausführungsformen kann das Gegenschraubelement durch ein gesondertes Bauteil gebildet sein. Zwecks Aufnahme des Schraubelements 300 weist die Meniskuskomponente 200 eine Längsbohrung 205 auf, die bei der gezeigten Ausführungsform mit einer Einführöffnung 206 versehen ist. Bei der gezeigten Ausführungsform dient die Einführöffnung 206 einem Einführen des Schraubelements 300.

Zum Anbringen der Meniskuskomponente 200 an der Tibiakomponente 100 wird zunächst der Gegenverbindungsabschnitt 202 mit dem Verbindungsabschnitt 102 verrastet. Hierdurch wird die besagte Fügeverbindung ausgebildet. Zur zusätzlichen Fixierung der Fügeverbindung wird das in die Längsbohrung 205 der Meniskuskomponente 200 eingesetzte Schraubelement 300 mit dem Gegenschraubelement 103 verschraubt.

Bei der gezeigten Ausführungsform ist das Schraubelement 300 eine Schraube S und das Gegenschraubelement 103 ist eine Gewindebohrung G. Eine solche Gestaltung ist jedoch nicht unbedingt erforderlich. Alternativ kann die Schraubverbindung nach Art eines Bajonettverschlusses gestaltet sein.

Die Meniskuskomponente 200 wird üblicherweise intraoperativ an der bereits an der Tibia befestigten Tibiakomponente 100 angebracht. Um auch während der Operation eine leichte Zugänglichkeit der Schraubverbindung zu gewährleisten, ist die Längsbohrung 205 relativ zu einer proximodistalen Längsachse 207 der Meniskuskomponente 200 in proximaler Richtung anterior geneigt. Mit anderen Worten ausgedrückt, ist eine Bohrungsachse 208 der Längsbohrung 205 derart schräg zu der proximodistalen Längsachse 207 orientiert, dass die Einführöffnung 206 an einer anterioren Vorderseite 209 der Meniskuskomponente 200 angeordnet ist.

In dem anhand Fig. 2 gezeigten befestigten Zustand ist die proximodistale Längsachse 207 koaxial zu einer proximodistalen Längsachse 104 der Tibiakomponente 100 ausgerichtet.

Die Gleitfläche 201 weist einen anterioren Rand 210 auf. Die Einführöffnung 206 ist relativ zu dem anterioren Rand 210 in distaler Richtung versetzt angeordnet. Durch die Anordnung der Einführöffnung 206 an der anterioren Vorderseite 209 und/oder unterhalb des anterioren Rands 210 wird eine besonders leichte Zugänglichkeit der Schraubverbindung erreicht.

Bei der gezeigten Ausführungsform ist die Längsbohrung 205 und/oder deren Bohrungsachse 208 in einer proximodistal und anteroposterior erstreckten Mittellängsebene der Kniegelenkendoprothese 1 angeordnet. Dabei ist die Längsbohrung 205 und/oder die Bohrungsachse 208 ausgehend von der proximodistalen Längsachse 207 vorliegend um etwa 60° nach vorne geneigt. Bei weiteren Ausführungsformen beträgt der Neigungswinkel beispielsweise zwischen 10° und 80°.

Bei der gezeigten Ausführungsform ist das Gegenschraubelement 103 in Form der Gewindebohrung G unmittelbar in die Tibiakomponente 100 eingebracht. Es versteht sich, dass die Gewindebohrung G koaxial zu der Längsbohrung 205 ausgerichtet ist. Unter Bezugnahme auf Fig. 2 bezeichnet die Bohrungsachse 208 sowohl die Längsachse der Längsbohrung 205 als auch die Längsachse der Gewindebohrung G. Insoweit gilt das zur Neigung der Längsbohrung 205 Gesagte, mutatis mutandis, auch für die Neigung der Gewindebohrung G sowie für die Neigung des Schraubelements 300. Bei der gezeigten Ausführungsform ist die Gewindebohrung G eine Durchgangsbohrung und erstreckt sich ausgehend von dem proximalen Verbindungsabschnitt 102 distal und posterior durch die Tibiakomponente 100, insbesondere durch deren Befestigungsabschnitt 101.

Im Übrigen weist die Schraube S eine dem Fachmann prinzipiell bekannte Gestaltung mit einem Kopfabschnitt 301, einem Schaftabschnitt 302 und einer Antriebsgeometrie 303 auf. Die Antriebsgeometrie 303 ist in den Kopfabschnitt 301 eingesenkt und kann auch als Werkzeugfläche bezeichnet werden. Die Antriebsgeometrie 303 ist zum Zusammenwirken mit einer komplementären Antriebsgeometrie eines Werkzeugs und/oder eines Werkzeugschafts eingerichtet. Der Schaftabschnitt 302 ist mit einem nicht näher bezeichneten Gewinde versehen, das komplementär zu der Gewindebohrung G ausgeführt und mit dieser verschraubbar ist. Unter Bezugnahme auf Fig. 2 ist der Kopfabschnitt 301 durch die Einführöffnung 206 zugänglich.

Bei der Ausführungsform nach den Fig, 1 und 2 weist die Kniegelenkendoprothese 1 zudem eine in die Meniskuskomponente 200, genauer deren Längsbohrung 205, eingesetzte Druckhülse 400 auf. Die Druckhülse 400 dient insbesondere einer mechanischen Verstärkung der Längsbohrung 205 und wirkt unmittelbar mit dem Schraubelement 300 zusammen. Die Meniskuskomponente 200 ist vorliegend aus einem polymeren Werkstoff gefertigt. Die Druckhülse 400 ist aus einem metallischen Werkstoff gefertigt. Es versteht sich, dass die Druckhülse 400 nicht unbedingt vorhanden sein muss. Dementsprechend weisen weitere Ausführungsformen keine solche Druckhülse auf.

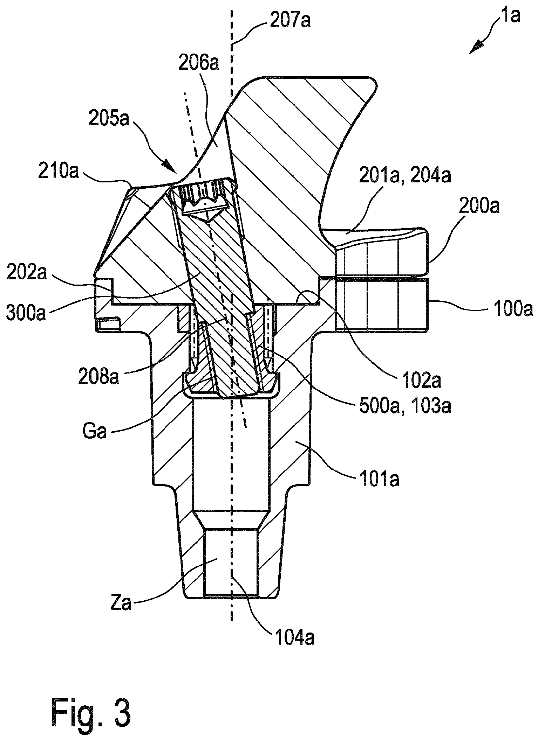

Anhand der Fig. 3 bis 6 sind weitere Ausführungsformen erfindungsgemäßer Kniegelenkendoprothesen 1a, 1b gezeigt. Zur Vermeidung von Wiederholungen werden nachfolgend lediglich wesentliche Unterschiede der Kniegelenkendoprothesen 1a, 1b gegenüber der Kniegelenkendoprothese 1 nach den Fig. 1 bis 2 im Detail erläutert. Das zuvor bereits zu der Kniegelenkendoprothese 1 nach den Fig. 1 und 2 Gesagte gilt, mutatis mutandis und sofern nicht Abweichendes beschrieben ist, auch für die Kniegelenkendoprothesen 1a, 1b.

Die Kniegelenkendoprothese 1a nach Fig. 3 unterscheidet sich im Wesentlichen dadurch von der Kniegelenkendoprothese 1 nach den Fig. 1 und 2, dass die Gewindebohrung Ga nicht unmittelbar in die Tibiakomponente 100a eingebracht ist. Stattdessen ist die Gewindebohrung Ga an einer Gewindehülse 500a ausgebildet. Die Gewindehülse 500a fungiert als Gegenschraubelement 103a. Die Gewindehülse 500a ist fest mit der Tibiakomponente 100a verbunden. Zu diesem Zweck ist die Gewindehülse 500a vorliegend in eine Zentralbohrung Za des Befestigungsabschnitts 101a eingebracht und fest mit dieser verbunden. Die Zentralbohrung Za ist koaxial zu der proximodistalen Längsachse 104a der Tibiakomponente 100a orientiert. Dementgegen ist die Gewindebohrung Ga schräg zu der proximodistalen Längsachse 104a ausgerichtet. Genauer ist die Gewindebohrung Ga auf die bereits in Zusammenhang mit der Kniegelenkendoprothese 1 nach den Fig. 1 und 2 erläuterte Weise anterior geneigt. Die Gewindehülse 500a erlaubt insbesondere eine vereinfachte Fertigung. Im Speziellen kann auf das Einbringen einer geneigten Bohrung unmittelbar in die Tibiakomponente 100a verzichtet werden.

Weiter im Unterschied zu der Kniegelenkendoprothese 1 nach den Fig. 1 und 2 weist die Kniegelenkendoprothese 1a keine Druckhülse 400 auf.

Die Längsbohrung 205a und/oder die Bohrungsachse 208a weist zudem einen geringeren Neigungswinkel auf. Dieser beträgt in etwa 10°. Durch die geringere anteriore Neigung ist die Einführöffnung 206a - im Unterschied zu der Ausführungsform nach den Fig. 1 und 2 - nicht unterhalb des anterioren Rands 210a der Gleitfläche 201a angeordnet.

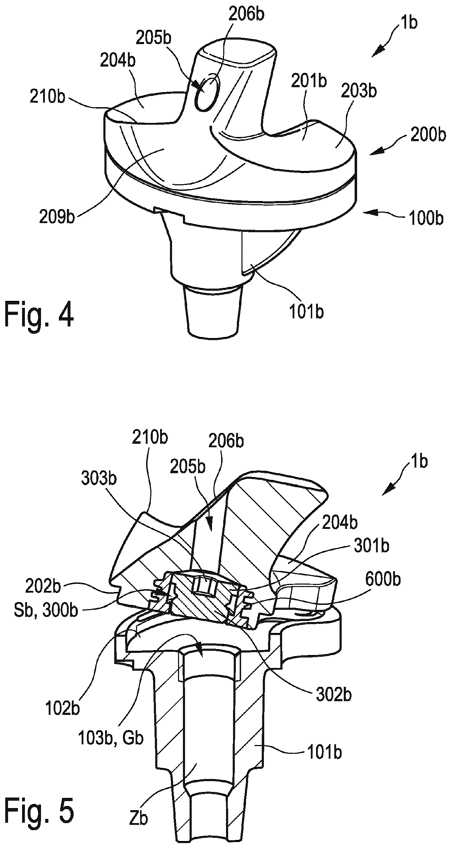

Die Kniegelenkendoprothese 1b nach den Fig. 4 bis 6 unterscheidet sich im Wesentlichen dadurch, dass das Schraubelement 300b - in einem von der Tibiakomponente 100b getrennten Zustand der Meniskuskomponente 200b - verliersicher in der Längsbohrung 205b gehalten ist. Hierdurch wird einem ungewollten Herausfallen des Schraubelements 300b aus der Längsbohrung 205b entgegengewirkt. Dies erlaubt eine einfache und sichere Handhabung der Meniskuskomponente 200 vor und während dem Anbringen an der Tibiakomponente 100b.

Zwecks verliersicherer Halterung ist das Schraubelement 300b radial und axial formschlüssig in der Längsbohrung 205b gehalten. Der Formschluss in axialer Richtung lässt vorliegend eine begrenzte Beweglichkeit des Schraubelements 300b zu.

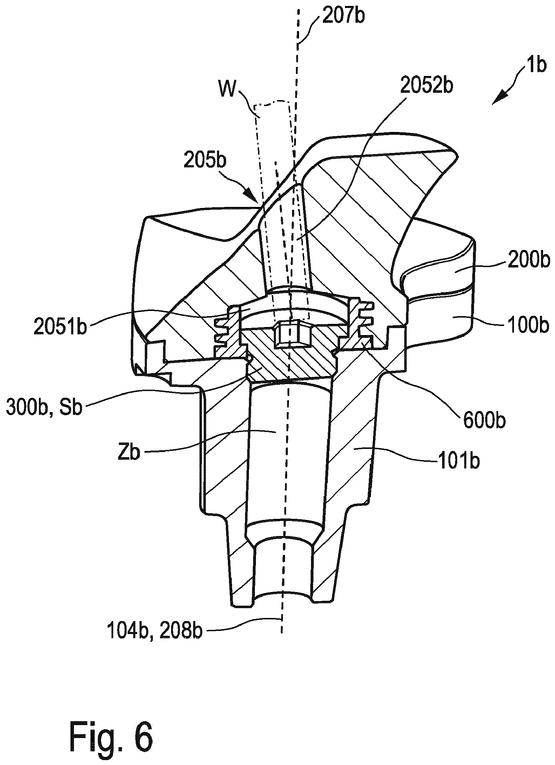

Zum Anbringen der Meniskuskomponente 200b an der Tibiakomponente 100b wird zunächst die bereits in Zusammenhang mit der Ausführungsform nach den Fig. 1 und 2 erläuterte Fügeverbindung zwischen dem Verbindungsabschnitt 102b und Gegenverbindungsabschnitt 202b ausgebildet. Hiernach wird das Schraubelement 300b mit dem Gegenschraubelement 103b verschraubt. Zu diesem Zweck wird ein Werkzeugschaft W, der in Fig. 6 schematisch angedeutet ist, durch die Einführöffnung 206b in die Längsbohrung 205b eingeführt und mit der Antriebsgeometrie 303b in Eingriff gebracht. Durch Drehen des Werkzeugschafts W wird eine Drehbewegung und ein Drehmoment auf das Schraubelement 300b übertragen und das Schraubelement 300b wird folglich mit dem Gegenschraubelement 103b verschraubt.

Bei der gezeigten Ausführungsform weist die Längsbohrung 205b einen ersten Bohrungsabschnitt 2051b und einen zweiten Bohrungsabschnitt 2052b auf (Fig. 6). Das Schraubelement 300b ist verliersicher in dem ersten Bohrungsabschnitt 2051b gehalten. Der zweite Bohrungsabschnitt 2052b weist einends die Einführöffnung 206b auf und mündet andernends in den ersten Bohrungsabschnitt 2051b. Dabei weisen die beiden Bohrungsabschnitte 2051b, 2052b unterschiedliche Durchmesser auf. Der Durchmesser (ohne Bezugszeichen) des zweiten Bohrungsabschnitts 2052b ist kleiner als der Durchmesser (ohne Bezugszeichen) des ersten Bohrungsabschnitts 2051b. Folglich weist auch die einends des zweiten Bohrungsabschnitts 2052b angeordnete Einführöffnung 206b einen kleineren Durchmesser als der erste Bohrungsabschnitt 2051b auf. Der Durchmesser des ersten Bohrungsabschnitts 2051b ist maßlich auf das aufzunehmende Schraubelement 300b abgestimmt und ist geringfügig größer als ein Außendurchmesser (ohne Bezugszeichen) des Kopfabschnitts 301b des Schraubelements 300b. Durch die in Relation zum Durchmesser des Schraubelements 300b verkleinerte Einführöffnung werden Irritationen des umgebenden Körpergewebes und damit einhergehende Beeinträchtigungen des Patienten vermieden.

Bei der gezeigten Ausführungsform beträgt der Durchmesser der Einführöffnung 206b und/oder des zweiten Bohrungsabschnitts 2052b in etwa 50 % des Durchmessers des ersten Bohrungsabschnitts 2051b.

Zur verliersicheren Halterung des Schraubelements 300b in der Längsbohrung 205b ist vorliegend eine Einsatzhülse 600b vorhanden. Die Einsatzhülse 600b bildet den ersten Bohrungsabschnitt 2051b. Mit anderen Worten ausgedrückt, kann auch davon gesprochen werden, dass der erste Bohrungsabschnitt 2051b mit der Einsatzhülse 600b versehen ist. Die Einsatzhülse 600b ist aus einem metallischen Werkstoff gefertigt und fest in die Meniskuskomponente 200b eingesetzt. Vorliegend ist die Einsatzhülse 600b in die polymere Meniskuskomponente 200b eingegossen. Eine solche Gestaltung ist jedoch nicht zwingend. Beispielsweise kann die Einsatzhülse 600b auch auf eine andere dem Fachmann bekannte Weise fest mit der Meniskuskomponente 200b verbunden sein.

Das Schraubelement 300b ist radial formschlüssig und axial begrenzt beweglich in der Einsatzhülse 600b gehalten. Zu diesem Zweck weist die Einsatzhülse 600b einen Radialbund 601b auf, der als distaler Anschlag für den Kopfabschnitt 301b des Schraubelements 300b fungiert. Der Radialbund 601b ist distal an der Einsatzhülse 600b angeordnet. Eine nicht näher bezeichnete Länge des Schaftabschnitts 302b ist derart bemessen, dass das daran befindliche Gewinde in einer distalen - durch den Radialbund 601b formschlüssig begrenzten - Endlage des Schraubelements 300b mit einer ausreichenden axialen Überdeckung mit dem Gegenschraubelement 103b verschraubbar ist.

Bei der Ausführungsform nach den Fig. 4 bis 6 ist das Gegenschraubelement 103b wiederum als Gewindebohrung Gb gestaltet. Die Gewindebohrung Gb ist unmittelbar in die Tibiakomponente 100b eingebracht und koaxial zu deren Zentralbohrung Zb. Die Zentralbohrung Zb ist durch den Befestigungsabschnitt 101b längserstreckt, als Durchgangsbohrung ausgeführt und weist einends - an ihrem proximalen Ende - die Gewindebohrung Gb auf. Die Gewindebohrung Gb - und damit auch das Schraubelement 300b - ist koaxial zu der proximodistalen Längsachse 104b. Folglich ist die Bohrungsachse 208b ebenfalls koaxial. Dies wenigstens im Bereich des ersten Bohrungsabschnitts 2051b. Der zweite Bohrungsabschnitt 2052b ist vorliegend geringfügig anterior geneigt (siehe Fig. 6). Die anteriore Neigung erlaubt wiederum eine verbesserte Zugänglichkeit der Einführöffnung 206b und ein vereinfachtes Einführen des Werkzeugschafts W zum Antreiben des Schraubelements 300b. Bei einer in den Figuren nicht gezeigten Ausführungsform ist die Längsbohrung nicht anterior geneigt und/oder parallel zu der proximodistalen Längsachse der Meniskuskomponente.

Es versteht sich, dass einzelne Merkmale der Kniegelenkendoprothesen 1, 1a, 1b zu weiteren Ausführungsformen miteinander kombiniert werden können. Beispielsweise können die Kniegelenkendoprothesen 1, 1a nach den Fig. 1 und 2 sowie 3 jeweils mit einer verliersicheren Halterung des Schraubelements versehen sein. Umgekehrt kann die Kniegelenkendoprothese 1b mit einer (vollständig und nicht lediglich abschnittsweise) anterior geneigten Längsbohrung ausgestattet werden.

## Patentansprüche

1. Kniegelenkendoprothese (1, 1a, 1b), aufweisend
eine Tibiakomponente (100, 100a, 100b) mit einem distalen Befestigungsabschnitt (101, 101a, 101b), der zur Befestigung an einer proximalen Tibia eingerichtet ist, und mit einem proximalen Verbindungsabschnitt (102, 102a, 102b), und aufweisend
eine Meniskuskomponente (200, 200a, 200b) mit einer proximalen Gleitfläche (201, 201a, 201b), die zur Artikulation mit einer femoralen Gelenkkomponente eingerichtet ist, und mit einem distalen Gegenverbindungsabschnitt (202, 202a, 202b), der zum Ausbilden einer Fügeverbindung mit dem proximalen Verbindungsabschnitt (102, 102a, 102b) der Tibiakomponente (100, 100a, 100b) eingerichtet ist,
**dadurch gekennzeichnet, dass** eine Schraubverbindung vorhanden und zur zusätzlichen Fixierung der Fügeverbindung eingerichtet ist, wobei die Schraubverbindung wenigstens ein in eine Längsbohrung (205, 205a, 205b) der Meniskuskomponente (200, 200a, 200b) eingesetztes Schraubelement (300, 300a, 300b) und wenigstens ein der Tibiakomponente (100, 100a, 100b) zugeordnetes Gegenschraubelement (103, 103a, 103b) aufweist, mit welchem das Schraubelement (300, 300a, 300b) zum Ausbilden der Schraubverbindung verschraubbar ist.

2. Kniegelenkendoprothese (1, 1a, 1b) nach Anspruch 1, **dadurch gekennzeichnet, dass** die Längsbohrung (205, 205a, 205b) relativ zu einer proximodistalen Längsachse (207, 207a, 207b) der Meniskuskomponente (200, 200a, 200b) in proximaler Richtung anterior geneigt ist.

3. Kniegelenkendoprothese (1, 1a, 1b) nach Anspruch 1 oder 2, **dadurch gekennzeichnet, dass** die Längsbohrung (205, 205a, 205b) eine Einführöffnung (206, 206a, 206b) zum Einführen des Schraubelements (300, 300a, 300b) und/oder eines Werkzeugschafts (W) zum Antreiben des Schraubelements (300, 300a, 300b) aufweist.

4. Kniegelenkendoprothese (1) nach Anspruch 3, **dadurch gekennzeichnet, dass** die Einführöffnung (206) an einer anterioren Vorderseite (209) der Meniskuskomponente (200) angeordnet ist.

5. Kniegelenkendoprothese (1) nach Anspruch 3 oder 4, **dadurch gekennzeichnet, dass** die Einführöffnung (206) relativ zu einem anterioren Rand (210) der Gleitfläche (201) in distaler Richtung nach unten versetzt angeordnet ist.

6. Kniegelenkendoprothese (1, 1a, 1b) nach einem der vorhergehenden Ansprüche, **dadurch gekennzeichnet, dass** das Schraubelement (300, 300a, 300b) in einem von der Tibiakomponente (100, 100a, 100b) getrennten Zustand der Meniskuskomponente (200, 200a, 200b) verliersicher in der Längsbohrung (205, 205a, 205b) gehalten ist.

7. Kniegelenkendoprothese (1b) nach Anspruch 6, **dadurch gekennzeichnet, dass** die Längsbohrung (205b) einen ersten Bohrungsabschnitt (2051b), in welchem das Schraubelement (300b) verliersicher gehalten ist, und einen zweiten Bohrungsabschnitt (2052b) aufweist, der einends die Einführöffnung (206b) aufweist und andernends in den ersten Bohrungsabschnitt (2051b) mündet.

8. Kniegelenkendoprothese (1b) nach Anspruch 7, **dadurch gekennzeichnet, dass** ein Durchmesser der Einführöffnung (206b) kleiner ist als ein Durchmesser des ersten Bohrungsabschnitts (2051b).

9. Kniegelenkendoprothese (1b) nach Anspruch 8, **dadurch gekennzeichnet, dass** der Durchmesser der Einführöffnung (206b) weniger als 80 %, bevorzugt weniger als 60 %, besonders bevorzugt weniger als 50 %, des Durchmessers des ersten Bohrungsabschnitts (2051b) beträgt.

10. Kniegelenkendoprothese (1b) nach einem der Ansprüche 7 bis 9, **dadurch gekennzeichnet, dass** der erste Bohrungsabschnitt (2051b) mit einer, insbesondere in die Meniskuskomponente (200b) eingegossenen und/oder metallischen, Einsatzhülse (600b) versehen ist, in welcher das Schraubelement (300b) radial und axial formschlüssig gehalten ist.

11. Kniegelenkendoprothese (1, 1a, 1b) nach einem der vorhergehenden Ansprüche, **dadurch gekennzeichnet, dass** das Gegenschraubelement (103, 103a, 103b) eine Gewindebohrung (G, Ga, Gb) ist.

12. Kniegelenkendoprothese (1, 1a, 1b) nach Anspruch 11, **dadurch gekennzeichnet, dass** die Gewindebohrung (G, Gb) in den Befestigungsabschnitt (101, 101b) der Tibiakomponente (1, 1b) eingebracht und/oder koaxial zu einer proximodistalen Längsachse (104b) der Tibiakomponente (100b) längserstreckt ist.

13. Kniegelenkendoprothese (1, 1a, 1b) nach Anspruch 11, **dadurch gekennzeichnet, dass** die Gewindebohrung (Ga) in eine in die Tibiakomponente (101a), insbesondere den Befestigungsabschnitt (101a), eingesetzte Gewindehülse (500a) eingebracht ist und/oder relativ zu einer proximodistalen Längsachse (104, 104a) der Tibiakomponente (100, 100a) geneigt ist.
